# EUROPÄISCHE PATENTSCHRIFT

(11) **EP 0 636 355 B1**
(45) Veröffentlichungstag und Bekanntmachung des Hinweises auf die Patenterteilung: **21.08.1996**
(21) Anmeldenummer: 94109908.7
(22) Anmeldetag: 27.06.1994
(51) Int. Cl.: A61F 6/08

(54) **Würfelpessar**
Cubic pessary
Pessaire cubique

(30) Priorität: 28.07.1993 DE 9311130 U; 01.10.1993 DE 9315520 U
(43) Veröffentlichungstag der Anmeldung: 01.02.1995
(73) Patentinhaber: Büttner-Frank GmbH, D-91058 Erlangen (DE)
(72) Erfinder: BÜTTNER-FRANK, Brigitte, D-91058 Erlangen (DE)
(74) Vertreter: Tergau, Enno, Dipl.-Ing.

(56) Entgegenhaltungen:
- WO-A-86/02826
- DE-A- 822 877
- FR-A- 2 484 247
- US-A- 1 604 511
- US-A- 3 780 730
- US-A- 4 922 928
- PSCHYREMBEL, Willibald: Klinisches Wörterbuch (1982), S. 912

## Beschreibung

Die Erfindung betrifft ein Pessar mit den Merkmalen des Oberbegriffs des Anspruches 1.

Ein Pessar dient zur Behandlung der Gebärmutter- und Scheidensenkung sowie zur Abstützung eines erschlafften Beckenbodens. Auch wird durch seinen Einsatz eine Lageverbesserung der Blase und des Mastdarmes erreicht.

Ein aus dem Prospekt "SILICON PESSARE" der Firma Büttner-Frank GmbH bekanntes Würfelpessar besteht aus Silikon-Elastomer. Die als Saugnäpfe wirksamen Vertiefungsmulden dienen zu seiner Applikation z.B. an der Blasenwand des Patienten. Die Lösung des Pessars aus seiner Saugstellung erfordert teilweise erhebliche Kräfte, die von außen über das Griffband durch den Patienten aufgebracht werden müssen. Dabei ist es bekannt, das Griffband nicht zentral, sondern im Bereich einer Ecke der Würfelform zu fixieren. Dies bewirkt - bezogen auf die virtuellen Würfeloberflächen - einen etwa diagonalen Kraftangriff, der beim Zug eine leichte Würfeldrehung erzeugt und dadurch den Lösevorgang erleichert.

Bei dem bekannten Pessar besteht das Griffband aus einem textilen Kunststoffaden ähnlich einem Nähfaden von vergleichsweise geringer Fadenstärke. Diese Griffbandausbildung hat eine Reihe von Nachteilen. Durch die geringe Fadenstärke entsteht im Scheidenbereich des Patienten eine Verletzungsgefahr durch Einschneiden. Zur Lösung des Pessars aus seiner Applikationsstellung müssen erhebliche Lösekräfte aufgebracht werden. Wegen der Unelastizität des Fixierfadens muß er eine erhebliche Länge aufweisen, um von der Patientin von außen ergriffen und zur Vermeidung eines Durchrutschens mehrmals um den Finger gewickelt werden zu können. Die notwendige Bandlänge ist unangenehm, weil das Griffband wegen seiner Überlänge aus der Scheide hinaushängt und nicht nach Art eines Tampons untergebracht werden kann.

Der Erfindung liegt die Aufgabe zugrunde, ein Pessar der eingangs genannten Art handhabungsfreundlich und körperangenehm zu gestalten. Diese Aufgabe ist durch die Merkmalskombination des Anspruchs 1 gelöst.

Erfindungsgemäß besteht das Griffband aus einem elastischen Werkstoff, insbesondere aus einem Silikon-Elastomer. Durch den elastischen Werkstoff ist es möglich, das Griffband von vornherein mit einer Länge auszustatten, die im Gebrauchszustand sein Heraushängen aus der Scheide verhindert. Die Fertigung aus Silikon-Elastomer hat den weiteren Vorteil, daß gerade dieser Werkstoff besonders körperverträglich ist. Schließlich ist gerade die Implantierbarkeit dieses Werkstoffes im medizinisch-therapeutischen Bereich allgemein bekannt (Deutsches Arzneibuch DAB 10, Grundfassung 1991, S. 1 und 2).

Ein weiterer Vorteil besteht darin, daß ein aus Silikon-Elastomer bestehendes Griffband zur Übertragung der notwendigen Zugkräfte unter Wirksamkeit der gewünschten Elastizität einen deutlich größeren Strangdurchmesser aufweist als das herkömmliche Griffband. Dadurch wird die Schnittgefahr bei der Benutzung verhindert. Außerdem verhindert die deutlich größere Strangstärke die Gefahr des Ausreißens des Fixierbereiches am Pessar. Zur Erhöhung der Ausreißfestigkeit im Fixierbereich sieht Anspruch 1 vor, den Werkstoff des Pessars im Durchdringungsbereich des Griffbandes härter einzustellen als im übrigen Körperbereich. Schließlich hat die deutlich größere Strangstärke den Vorteil, daß das Griffband als endlose Ringschlinge dadurch ausgebildet werden kann, daß seine Enden stoffschlüssig, z.B. durch Verklebung zu einer Ringschlinge miteinander verbindbar sind. Wenn diese Ringschlinge in etwa ihre Ringform beibehaltender Position am würfelförmigen Pessar fixiert ist, so behält sie aufgrund der ihr innewohnenden Rückstellkraft ihre Ausgangs-Ringform bei. Dadurch verbleibt die Ringschlinge im Tragezustand ohne weitere Vorkehrungen innerhalb der Scheide, obwohl sie unter Zug für das Lösen des Pessars eine ausreichende, außerhalb der Scheide liegende Grifflänge zur Verfügung stellt. Diese vorteilhafte Wirkung wird in Kombination mit der gewünschten Erleichterung der Lösbarkeit des Pessars durch das Kennzeichnungsmerkmal des Anspruches 5 erleichtert. Hierdurch ist eine Applikationsform des Griffbandes möglich, die die Ringschlinge im Fixierbereich auf einer einem Kreisbogen optimal angenäherten Wegstrecke führt und daher den gewünschten leichten Rückstelldruck begünstigt, der die Ringschlinge in ihrem außerhalb des Pessars liegenden Bereich in eine ringähnliche Kreisform drängt und damit im Tragezustand eine von außen verdeckte Unterbringung des Griffbandes ermöglicht.

Von besonderem Vorteil ist eine Werkstoffgleiche von Pessar und Griffband, zweckmäßig beide aus Silikon-Elastomer. Durch seine Eigenelastizität ist das Pessar auch beim Wasserlassen und Stuhlgang nicht hinderlich, da es elastisch zusammengedrückt wird und sich dadurch den Organen anpaßt.

Gemäß den Ansprüchen 11 bis 13 wird ein gelochtes Würfelpessar vorgeschlagen. Es ist vorteilhaft nach der Entbindung und nach Operationen einsetzbar. Sekret bzw. Noch-Blutung sowie Wundfluß können durch die Lochung hindurch ablaufen.

Die Erfindung wird anhand der in den Figuren dargestellten Ausführungsbeispiele näher erläutert. Es zeigen:
- Fig.1: eine perspektivische Darstellung des Würfelpessars in einer ersten Ausführungsform.
- Fig.2: die perspektivische Darstellung des Würfelpessars in einer zweiten Ausführungsform.

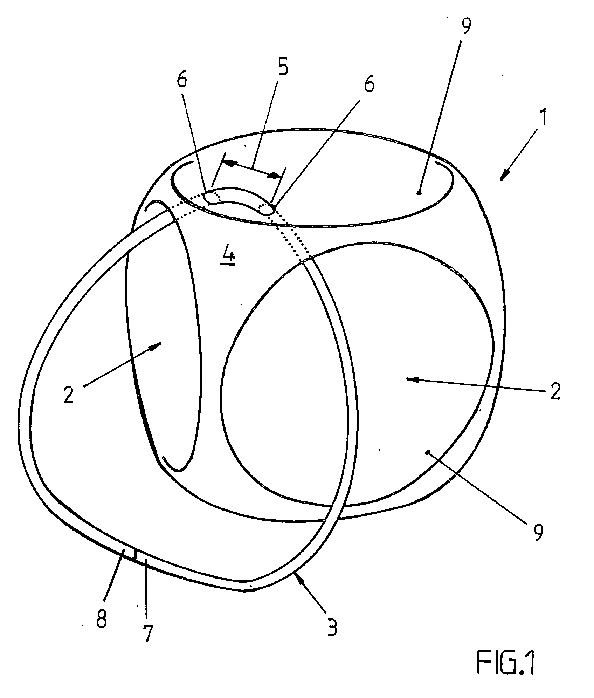

Das Pessar 1 in Fig.1 weist die Umrißform etwa eines Würfels mit seine Flächen etwa ausfüllenden Saugnäpfen in Form von zur Würfelmitte hin ausgemuldeten Vertiefungen 2 auf. Das Pessar 1 ist darüber hinaus mit einem Griffband 3 in Form einer Griffschlinge versehen. Sie dient zur Entfernung des Pessars aus seiner Applikationsstellung beim Patienten. Das Griffband 3 durchsetzt zu seiner Fixierung am Pessar 1 mindestens eine Wand des Würfels. Beim Ausführungsbeispiel erfolgt dies im Bereich der Würfelecke 4. Dabei durchsetzt das Griffband 3 zweimal eine zwischen zwei benachbarten Vertiefungsmulden 2 befindliche Würfelwand. Das Griffband 3 durchsetzt dabei sämtliche drei der Würfelecke 4 benachbarten Vertiefungsmulden 2, wobei im Ausführungsbeispiel die obere Vertiefungsmulde 2 zwei einen Abstand 5 voneinander einhaltende Durchsatzöffnungen 6 aufweist, während im Bereich der beiden anderen der Würfelecke 4 zugeordneten Vertiefungsmulden 2 nur jeweils eine hier nicht näher dargestellte Durchsatzöffnung 6 vorhanden ist. Im Bereich der oberen Vertiefungsmulde 2 liegt das Griffband 3 zwischen den beiden Durchsatzöffnungen 6 über die Länge des Abstandes 5 frei.

Das Griffband 3 ist als endlose Ringschlinge ausgebildet, die durch eine stoffschlüssige Verbindung, insbesondere durch eine Verklebung der beiden Enden 7, 8 des Griffbandes 3 hergestellt ist. Das Griffband 3 weist eine Bandstärke, insbesondere einen Durchmesser von 1,5 bis 3 mm auf. Der durch das Griffband 3 etwa gebildete Ring weist einen Ringdurchmesser von ca. 5-6 cm auf, der im Gebrauchszustand eine ausreichende Grifflänge für die Lösung des Pessars aus seiner Applikationsstellung zur Verfügung stellt.

Das Griffband 3 besteht aus demselben Silikon-Elastomer wie das in Würfelform vorliegende Pessar 1. Der Werkstoff des in Form eines Würfels vorliegenden Pessars 1 ist im Durchdringungsbereich des Griffbandes 3, also beim Ausführungsbeispiel im Bereich der Würfelecke 4 härter eingestellt als im übrigen Würfelbereich.

Die Mantelflächen der Vertiefungsmulden 2 haben in an sich bekannter Weise die Form eines Kegelmantels, dessen Kegelspitze bis nahe an die Würfelmitte reicht und dessen Kegelachse lotrecht zu den virtuellen Würfelflächen verläuft.

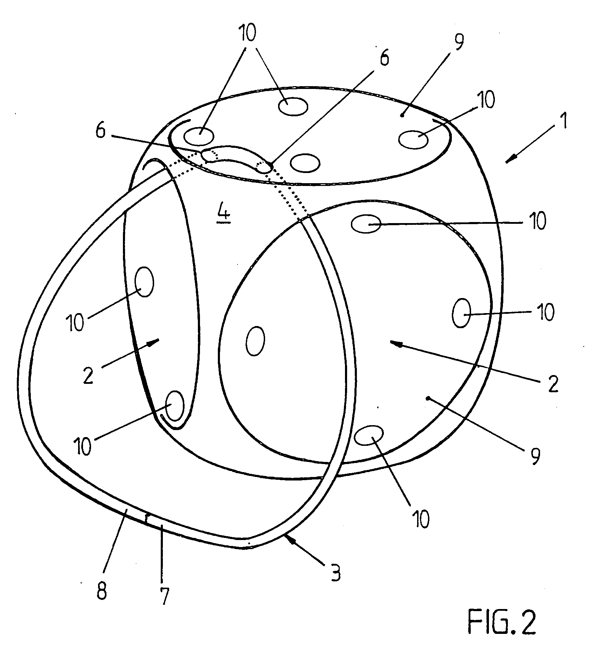

Das Pessar 1 gemäß Fig.2 unterscheidet sich vom Ausführungsbeispiel gemäß Fig.1 lediglich dadurch, daß die Randbereiche 9 der einzelnen Vertiefungsmulden 2 von Lochungen 10 durchsetzt sind. Jede Vertiefungsmulde 2 weist insgesamt 4 Lochungen 10 auf. Dabei durchsetzt jede Lochung 10 die Würfelwand zwischen zwei benachbarten Randbereichen 9 zweier benachbarter Vertiefungsmulden 2. Die Lochung 10 weist eine kreisförmige Querschnittsfläche auf. Andere, z.B. elliptische Querschnittsflächen sind ebenfalls denkbar.

Zur Lösung des Pessars aus seiner Applikationsstellung beim Patienten wird das Griffband 3 ergriffen, indem der Finger des Patienten in die Ringschlinge hineingreift und an dieser zieht. Dadurch verformen sich die beiden einander gegenüberliegenden freien Schlingenbereiche zu zwei etwa parallel zueinander verlaufenden Strängen unter elastischer Dehnung. Diese Verformung und Dehnung gewährleisten eine ausreichende Grifflänge in einer Position, in der sich die für die Lösung des Pessars erforderliche Zugkraft aufbaut. Der Zugkraftangriff am Pessar gestaltet sich besonders weich und damit schonend.

### Bezugszeichenliste

- 1: Pessar
- 2: Vertiefungsmulde
- 3: Griffband
- 4: Würfelecke
- 5: Abstand
- 6: Durchsatzöffnung
- 7: Ende
- 8: Ende
- 9: Randbereich
- 10: Lochung

## Patentansprüche

1. Pessar (1) in Form etwa eines Würfels aus elastischem Werkstoff
- mit seine Flächen etwa ausfüllenden Saugnäpfen in Form von zur Würfelmitte hin ausgemuldeten Vertiefungen (2) und
- mit einem zu seiner Entfernung aus der Applikationsstellung dienenden und an einer Würfelwand fixierten Griffband (3), insbesondere einer Griffschlinge,
dadurch gekennzeichnet,
- daß das Griffband (3) aus einem elastischen Werkstoff, insbesondere aus einem Silikon-Elastomer besteht und
- daß der Werkstoff des Pessars (1) im Fixierbereich des Griffbandes (3) härter eingestellt ist als im übrigen Würfelbereich.

2. Pessar nach Anspruch 1,
dadurch gekennzeichnet,
daß das Griffband (3) die Würfelwand zwischen benachbarten Randbereichen (9) zweier benachbarter Vertiefungsmulden (2) durchsetzt.

3. Pessar nach Anspruch 1 oder 2,
dadurch gekennzeichnet,
daß das Griffband (3) im Bereich einer Würfelecke (4) mindestens eine Würfelwand durchsetzt.

4. Pessar nach einem oder mehreren der vorhergehenden Ansprüche,
dadurch gekennzeichnet
daß das Griffband (3) drei einer gemeinsamen Würfelecke (4) zugeordnete Vertiefungsmulden (2) durchsetzt.

5. Pessar nach Anspruch 4,
dadurch gekennzeichnet,
daß die mittlere der drei Vertiefungsmulden (2) zwei mit Abstand zwischen sich benachbarte Durchsatzöffnungen (6) für das Griffband (3) aufweist.

6. Pessar nach einem oder mehreren der vorhergehenden Ansprüche,
dadurch gekennzeichnet,
daß das Griffband (3) als endlose Ringschlinge ausgebildet ist.

7. Pessar nach Anspruch 6,
dadurch gekennzeichnet,
daß die Ringschlinge durch stoffschlüssige Verbindung, insbesondere durch Verklebung der Enden (7,8) des Griffbandes (3) hergestellt ist.

8. Pessar nach einem oder mehreren der vorhergehenden Ansprüche,
dadurch gekennzeichnet,
daß das Griffband (3) eine Bandstärke, insbesondere einen Querschnittsdurchmesser von 1,5 bis 3 mm aufweist.

9. Pessar nach einem oder mehreren der vorhergehenden Ansprüche,
gekennzeichnet durch
einen Ringschlingen-Durchmesser von 5 bis 6 cm.

10. Pessar nach einem oder mehreren der vorhergehenden Ansprüche,
dadurch gekennzeichnet,
daß er aus demselben Silikon-Elastomer besteht wie das Griffband.

11. Pessar nach einem oder mehreren der vorhergehenden Ansprüche,
dadurch gekennzeichnet,
daß er für den Durchtritt von Flüssigkeit durchlocht ist.

12. Pessar nach Anspruch 11,
dadurch gekennzeichnet,
daß die Lochung (10) die Würfelwand zwischen benachbarten Randbereichen (9) zweier benachbarter Vertiefungsmulden (2) durchsetzt.

13. Pessar nach Anspruch 12,
dadurch gekennzeichnet,
daß jede Würfelwand mindestens eine Lochung (10) aufweist.

## Claims

1. Pessary (1) in the approximate form of a cube made of elastic material
- with absorbent cups which more or less fill its surfaces and which are in the form of depressions (2) which have been hollowed out towards the centre of the cube, and
- with a grip band (3), in particular a grip loop, which is fixed on a cube wall and is used for removing the pessary from the application site,
characterized in that
- the grip band (3) consists of an elastic material, in particular of a silicone elastomer, and
- the material of the pessary (1) in the area of attachment of the grip band (3) is harder than in the remaining area of the cube.

2. Pessary according to Claim 1, characterized in that the grip band (3) passes through the cube wall between adjoining edge areas (9) of two adjacent hollowed depressions (2).

3. Pessary according to Claim 1 or 2, characterized in that the grip band (3) passes through at least one cube wall in the area of a cube corner (4).

4. Pessary according to one or more of the preceding claims, characterized in that the grip band (3) passes through three hollowed depressions (2) assigned to a common cube corner (4) .

5. Pessary according to Claim 4, characterized in that the central of the three hollowed depressions (2) has two through-openings (6), adjacent to each other at a distance, for the grip band (3).

6. Pessary according to one or more of the preceding claims, characterized in that the grip band (3) is designed as an endless annular loop.

7. Pessary according to Claim 6, characterized in that the annular loop is produced by integral connection, in particular by bonding together the ends (7, 8) of the grip band (3).

8. Pessary according to one or more of the preceding claims, characterized in that the grip band (3) has a band thickness, particularly a transverse diameter, of 1.5 to 3 mm.

9. Pessary according to one or more of the preceding claims, characterized by an annular loop diameter of 5 to 6 cm.

10. Pessary according to one or more of the preceding claims, characterized in that it consists of the same silicone elastomer as the grip band.

11. Pessary according to one or more of the preceding claims, characterized in that it is perforated for the passage of fluid.

12. Pessary according to Claim 11, characterized in that the perforation (10) passes through the cube wall between adjacent edge areas (9) of two adjacent hollowed depressions (2).

13. Pessary according to Claim 12, characterized in that each cube wall has at least one perforation (10).

## Revendications

1. Pessaire (1) ayant la forme approximative d'un cube, fait d'une matière élastique,
- présentant des ventouses qui couvrent à peu près totalement ses faces et sont constituées par des alvéoles (2) creusés en direction du centre du cube, et
- comprenant un cordon de poignée (3), notamment une boucle de cordon, servant à l'extraire de sa position d'application et fixé à une paroi du cube,
caractérisé
en ce que le cordon de poignée (3) est fait d'une matière élastique, notamment d'un élastomère de silicone, et
en ce que la matière du pessaire (1) est plus dure dans la région de fixation du cordon de poignée (3) que dans le reste du cube.

2. Pessaire selon la revendication 1,
caractérisé
en ce que le cordon de poignée (3) traverse la paroi du cube entre des régions marginales adjacentes (9) de deux alvéoles (2) adjacents.

3. Pessaire selon la revendication 1 ou 2,
caractérisé
en ce que le cordon de poignée (3) traverse au moins une paroi du cube dans la région d'un angle (4) du cube.

4. Pessaire selon une ou plusieurs des revendications précédentes,
caractérisé
en ce que le cordon de poignée (3) traverse trois alvéoles (2) adjacents à un même angle (4) du cube.

5. Pessaire selon la revendication 4,
caractérisé
en ce que celui des trois alvéoles (2) qui est en position centrale présente deux ouvertures traversantes (6), adjacentes séparées par un certain écartement, pour le passage du cordon de poignée (3).

6. Pessaire selon une ou plusieurs des revendications précédentes,
caractérisé
en ce que le cordon de poignée (3) est constitué par une bouche annulaire sans fin.

7. Pessaire selon la revendication 6,
caractérisé
en ce que la bouche annulaire est fabriquée par un assemblage par continuité de matière, en particulier par collage des extrémités (7, 8) du cordon de poignée (3).

8. Pessaire selon une ou plusieurs des revendications précédentes,
caractérisé
en ce que le cordon de poignée (3) présente une épaisseur de cordon, en particulier un diamètre de section, de 1,5 à 3 mm.

9. Pessaire selon une ou plusieurs des revendications précédentes,
caractérisé
par un diamètre de boucle annulaire de 5 à 6 cm.

10. Pessaire selon une ou plusieurs des revendications précédentes,
caractérisé
en ce qu'il est composé du même élastomère de silicone que le cordon de poignée.

11. Pessaire selon une ou plusieurs des revendications précédentes,
caractérisé
en ce qu'il est perforé pour donner passage à un liquide.

12. Pessaire selon la revendication 11,
caractérisé
en ce que la perforation (10) traverse la paroi du cube entre des régions marginales adjacentes (9) de deux alvéoles (2) adjacents.

13. Pessaire selon la revendication 12,
caractérisé
en ce que chaque paroi du cube présente au moins une perforation (10).
